Europäisches Patentamt

European Patent Office

Office européen des brevets

(11) Publication number: **0 249 359**
**A2**

# EUROPEAN PATENT APPLICATION

(21) Application number: **87304671.8**

(22) Date of filing: **27.05.87**

(51) Int. Cl.⁴: **C07C 51/295**

(30) Priority: **12.06.86 US 873580**

(43) Date of publication of application:
**16.12.87 Bulletin 87/51**

(84) Designated Contracting States:
**BE CH DE ES FR GB GR IT LI NL**

(71) Applicant: **STAUFFER CHEMICAL COMPANY**

**Westport Connecticut 06881(US)**

(72) Inventor: **Mirviss, Stanley Burton**
**90 Surrey Road**
**Stamford Connecticut 06903(US)**

(74) Representative: **Smith, Sydney et al**
**Elkington and Fife High Holborn House 52/54**
**High Holborn**
**London WC1V 6SH(GB)**

(54) **Process for production of fatty acids.**

(57) Straight or branch chain primary alcohols of from C-4 to C-30 chain lengths per molecule, or mixtures of branch and straight chains, are selectively reacted with an anhydrous alkali metal hydroxide. In the reaction, the temperature of reaction is gradually increased to maintain the reaction medium in a liquid non-viscous state. The reaction temperature is, however, maintained at a sufficiently low temperature to minimize by-product formation. The process is conducted at temperatures of from about 175°C to about 400°C for sufficient time to form the sodium salt of the acid which is then converted to the acid utilizing a mineral acid.

EP 0 249 359 A2

## PROCESS FOR PRODUCTION OF FATTY ACIDS

The invention relates to an improved process for preparing fatty acids by oxidative dehydrogenation.

The oxidative dehydrogenation of alcohols in the presence of an alkali metal hydroxide to produce the salt of the corresponding organic acid is known. The process, however, suffers from foaming; the necessity of redissolving the soap after preparation with water prior to acidification; the difficulty of removing the solid soaps after reaction from the reactor; and the necessity of steam distillation to separate the soap from the unreacted alcohol, all of which limit the yield and purity of the product obtained.

U.S. Patent No. 3,370,074 discloses a process for the preparation of carboxylic acid salts. The process disclosed is the oxidative dehydrogenation of at least one oxygen-containing compound selected from the group consisting of primary alcohols, ethers having at least two hydrogen atoms on at least one of the carbon atoms adjacent to the ether oxygen atom, aldehydes, aldols, and esters by a process which comprises reacting under oxidative dehydrogenation conditions in the liquid phase, a mixture consisting essentially of the oxygen-containing compound reactant, a substantially anhydrous alkali metal compound selected from the group consisting of alkali metal oxides and hydroxides, and between 0.5 and 8 mole percent water based on the alkali metal compound employed. The range specified for the alkali metal compound to the oxygen-containing compound reactant varied between 0.5:1 and 4:1, and preferably between 1:1 and 1.5:1. Recovery of the salt of the acid and formation of the acid itself was accomplished by adding an aqueous mineral acid to the hot liquid organic acid salt before solidification occurred.

U.S. Patent No. 3,560,537 discloses a process for producing straight chain monobasic carboxylic acid soaps and their derivatives. The process disclosed is the reaction of an alkali metal hydroxide with normal alcohols of from about 6 to about 30 carbon atoms per molecule in the presence of less reactive branched primary alcohols. In the presence of the less reactive branched primary alcohol, a carboxylic acid soap of a predominantly straight chain carbon skeleton configuration is produced in addition to hydrogen. There is further disclosed the selectivity of reaction of straight chain alcohols and freedom from methylene group attack. This is enhanced by using proper elevated temperatures in combination with a deficiency of alkali metal hydroxide, based on stoichiometric proportions for the total alcohol content of the reaction system, and in the absence of oxidants of methylene groups at the temperatures involved.

The object of the instant invention is the formation of fatty acids in high yields with high purity. Another object of the invention is to minimize process deficiencies such as foaming. A further object of the invention is to dispense with the need for dissolving the salt of the acid after formation and stripping to remove the unreacted alcohol therefrom.

## SUMMARY OF THE INVENTION

The invention is a process of preparing fatty acids which comprises reacting an oxygen-containing compound with an alkali metal hydroxide at a temperature necessary to complete the reaction, and wherein the temperature is continuously adjusted to maintain the reactants in a non-viscous liquid state to substantially eliminate foaming and produce the salt of a fatty acid. The invention further comprises contacting the salt of the fatty acid, preferably in a molten state, with a mineral acid to produce the fatty acid.

## DETAILED DESCRIPTION OF THE INVENTION

In accordance with the invention, high yields of the fatty acids are obtained by the oxidative-dehydrogenation of an oxygen-containing compound, preferably straight or branched chained alcohols, in the absence of a catalyst. The reaction is conducted with or without pressure at a temperature sufficiently high to complete the reaction but avoid by-product formation. The temperature during the process is continuously adjusted to maintain a liquid non-viscous state and to substantially eliminate foaming. An excess of alkali metal hydroxide over stoichiometric quantities is desirably maintained for the process to insure complete reaction of the oxygen-containing compound.

In the practice of the invention, oxygen-containing organic compounds and, more particularly, compounds having an oxygen atom attached to a carbon atom having at least two substituent hydrogen atoms are reacted with an alkali metal hydroxide. Preferably, in the practice of the invention, straight and/or branched chain primary alcohols, glycols, esters, ethers or aldehydes, are reacted with an alkali metal hydroxide at a temperature and pressure necessary to produce substantially pure acid products in high yields. By "primary alcohol" is meant any compound containing an hydroxyl group directly connected to a carbon atom having at least two substituent hydrogen group atoms. These oxygen-containing compounds can have from 1 to about 40 carbon atoms per molecule. The preferred oxygen-containing organic compounds are those having from about 4 to about 24 carbon atoms per molecule. It is understood that the oxygen-containing compound reactant defined above may be polyfunctional, if desired, and can include compounds such as, for example, glycols, where at least one of the two hydroxyl groups is a primary alcohols group. The alcohol can comprise a mixture of straight and branched chain primary alcohols, straight and branched chain aldehydes, and if desired, a mixture of primary alcohols and aldehydes, or esters together with aldols and ethers as defined above. Examples of suitable oxygen-containing compounds which can be used in a process of this invention include: n-butanol, n-octanol, n-dodecanol, n-eicosanol, n-heptanol, n-nonanol, 2-ethylhexanol, 2-methylbutanol, 2-methylhexanol, 2,4,4-trimethylpentanol-1, 2-n-hexyldecanol, n-octadecanol, 2-phenylethanol, 2-phenylpropanol, n-butyraldehyde, 2-ethylhexanal, 3,5,5-trimethylhexanol-1, 2,2,4,4-tetramethylpentanol-1, 2,2-dimethylpropanol, 2-ethylhexyl 2-ethylhexanoate, n-butyl n-hexanoate, n-octyl acetate, 2-methylhexyl isobutyrate, 2,4,4-trimethylpentanediol-1,3, hexanediol-1,4, di-n-pentylether, di-n-butyl ether, 2(n-butoxy) ethylalcohol, 2(beta-phenylethoxy)ethoxyethanol, 1,4-cyclohexanedimethanol, cyclopropylmethanol, neopentylglycol, 2,2,4-trimethylpentanediol-1,3 monoisobutyrate, and the like. Alcohols are the most preferred oxygen-containing compounds for practicing the invention.

The alkali metal compound of this reaction is preferably sodium hydroxide; others include potassium hydroxide. The amount of alkali metal compound utilized in the process of the invention can be from about 0% to about 100% over stoichiometric amounts, and desirably from about 2% to about 50% in excess of stoichiometric quantities. A 3% to 20% excess is preferred.

Although minor amounts of water may be added to the reactants, in the practice of the invention, it is proposed to add no water. The addition of water to the process was found to increase the potential foaming and expansion during the reaction. Water added during the reaction complicates the reaction by slowing the reaction which causes foaming problems. In addition, the use of added water would require the addition of a condenser to the reaction system. It was found advantageous in fact, to remove some of the water formed during the reaction, by venting with the hydrogen to maintain pressure.

The process is generally conducted at temperatures from about 175°C to about 400°C depending on the specific oxygen-containing compound being reacted. The preferred temperature range, however, is generally in the range of from about 275°C to about 370°C for completing the reaction, particularly with straight chain oxygen-containing reactants. The particular temperature schedule utilized varies with the particular oxygen-containing compound reactant, or mixtures thereof, due to differences in the melting points of the soap products of the reaction of the oxygen-containing compounds. A lower temperature is generally used at the beginning of the reaction process than at the end of the reaction since the oxygen-containing compounds are liquids at the temperature at which the reaction commences. As the reaction proceeds, more soap (or salt of the fatty carboxylic acid) forms, and less oxygen-containing compound is present. Since the soaps generally have much higher melting points than, for instance, alcohols and are not completely soluble at all proportions in the alcohols, the reaction mixture becomes viscous and semi-solid or pasty and, hence, difficult to stir. Concomitantly, the hydrogen gas by-product does not readily disengage from the reaction mixture into the free space above. As a result, foaming and reaction mixture volume expansion generally occurs. This can have disastrous effects. The foam may rise and/or the reaction mixture volume expansion may rise into the top, cooler parts of the reactor and solidify. The solids formed can plug off reactor outlets to overpressure relief or blow out devices, to the pressure gauge or to the hydrogen gas vent. An explosion could result.

It has been found that by gradually raising the temperature during the reaction according to a schedule predetermined to maintain a low viscosity, molten (fluid) condition and by avoiding too high temperatures which cause side reactions, foaming and excessive reaction mixture volume expansion can be substantially prevented. Temperatures to be utilized can be readily predetermined by determining the melting points of the product soap and mixtures of the soap with the raw material oxygen-containing compound. The soaps were found to be very fluid (low viscosity) liquids at only 5°C or more above their melting point.

It is preferred to use a low enough temperature during approximately the first half of the reaction to prevent side reactions from occurring. However, the temperature must, of course, be high enough for the reaction mixture to be liquid and non-viscous and for the reaction to proceed at a practical rate. Side reactions can result in the formation of dimer alcohols which are the major reaction side products of the process. The particular temperatures at the first half of the reaction depends on the particular oxygen-containing compound or mixtures used.

The temperatures during the second half of the reaction are most critical and must be increased as the reaction proceeds so that a top or maximum temperature is obtained well before the end of the reaction. This maximum temperature should be above the melting point of the product soap. Desirably, the maximum temperature should be at least 5°C above the melting point. Temperatures in excess of 20-50°C above this melting point may cause appreciable dimerization side reaction (Guerbet reaction) leading to formation of undesired dimeric acid impurities. This is particularly true with straight chain alcohols at 400°C and higher. The final or maximum temperature is lower for branched acids than for straight chain acids. Also, the final temperature is higher for $C_{10}$ and higher acids than for shorter chain lengths. Mixtures of soap may have somewhat lower melting points than any of the single soap mixture components, and so mixtures of alcohols will use somewhat lower final reaction temperatures than any of the single alcohol components. This is especially true for straight chain alcohol mixtures as opposed to single straight chain alcohols. At the higher temperatures required to maintain a molten fluid condition during the later phases of the reaction, temperatures of about 275°C for all branched chain alcohols and of about 370°C for all straight chain alcohols are desirable. Mixtures of the two types will utilize intermediate temperatures. A temperature - schedule as provided above provides essentially complete conversion, thus a catalyst is not needed.

The reaction can be conducted without pressure by venting all the hydrogen gas (maintaining an open system). It is, however, preferable to conduct the reaction under pressure. The pressure is autogenous and can range from 1 atmosphere to 100 atmospheres (14.7-1470 psig), with a range of 2 atmospheres to 30 atmospheres being preferred (29.4-441 psig). The pressure is generally controlled by venting the hydrogen gas by-product.

Agitation is necessary during the reaction process and is especially so for straight chain alcohols. Agitation should preferably be vigorous to release the hydrogen gas from the reaction liquid and for thorough dispersion of the alkali metal hydroxide. Reactors designed for easy hydrogen gas escape for the liquid phase are beneficial.

The alkali metal hydroxide can be added in any form, preferably in an anhydrous condition. It is preferred to add it as a flake, or as a fine granular bead or powder to allow for good dispersion in the molten alcohol. Good dispersion is insured if the alkali metal hydroxide is added to the molten oxygen-containing compound in the reaction. Potassium hydroxide can be used in place of sodium hydroxide or a mixture of potassium hydroxide and sodium hydroxide. However, by-products are more likely to form when potassium hydroxide is utilized.

A catalyst is not utilized in the process of the invention so as to avoid by-product formation, especially dimeric acid products. It was found that the formation of these dimeric acids was enhanced when catalysts, especially metal oxides of Group IIB of the Periodic Table, (e.g., zinc oxide), or hydroxides are present. With the temperature schedule utilized in the practice of the invention, the reaction temperature is high enough for the rate of reaction to be substantially the same as that when a catalyst is used.

The reaction product consisting of the salt or salts of the acids is maintained in a molten or liquid condition after completion of the reaction while the molten soap is gradually added to the mineral acid for acidification to the corresponding fatty acids. The molten soap is preferably added quickly to the mineral acid with agitation. Little cooling is needed and there is substantially no violent reaction of the molten soap with the much cooler acid occurs. Addition of the mineral acid to the molten soap, as described in U.S. 3,370,074 discussed above, was found to cause a violent reaction with much steam evolution, spattering and soap solidification. The sodium salt of mineral acid tends to crust over the soap and the physical mixing of mineral acid with soap became extremely difficult. The preferred mode of addition proceeds smoothly without any mixing or solidification problems whatsoever. The fatty acid formed is essentially free of alcohol impurities. Hence, it is suitable as is for many purposes, including esterification or conversion to heavy metal salts without need for purification. For some purposes, it may be necessary to remove a small amount of water.

The following examples are descriptive embodiments of the invention but are not considered to be limiting of the scope of the invention. Examples I, II, and III were laboratory scale methods which, due to equipment limitations, prevented the addition of the salt of the soap in a molten state to the mineral acid. Example IV, which constitutes an experimental pilot plant scale run, exemplifies this feature of the invention.

## VAPOR PHASE CHROMATOGRAPHY ANALYSIS TECHNIQUE

The alcohol-acid product and dimer acid by-product content of the final product was determined by vapor phase chromatography (VPC) for the Examples provided.

The VPC analytical technique was used during the experimental runs and was based on the silylation of both starting alcohols and the product carboxylic acids. Typically 0.05 milliliter of either the acid, alcohol, or mixture of the two was added to 0.5 ml of bis-(trimethylsilyl) trifluoroacetamide containing 1% trimethylsilylchloride. After a few minutes standing, with occasional shaking, the sample was ready for use.

Normally 1 microliter of the silylated sample was then injected onto a 10% SE 30 on Chromosorb W, 6 ft. x 1/8 in. column. The injection port temperature was at 295°C. The helium flow rate was 21.0 ml per minute. Initially, the column was at 95°C. Five minutes after injection it was programmed to rise 8°C/min.

### EXAMPLE I

A 1-liter magnetic drive stirred Inconel autoclave was charged at room temperature with 484.7 g (3.35 moles) Monsanto's 7911 alcohol (assumed molecular wt. 144.6 gm) and 252.6 g (3.82 moles) flake NaOH. The Monsanto 7911 alcohol is a combination of straight and branched $C_7$, $C_9$ and $C_{11}$ primary alcohols which is 1/3 branched. The branching is mostly methyl with some ethyl branching at the alpha carbon. The autoclave was stirred at 300 rpm. The reactor was first flushed with nitrogen and then electrically heated with stirring. There was no evidence of any foaming during the runs. A thermocouple probe at the 90% full level and a sight glass just above the reactor but below the condenser did not show any foaming or expansion of the reactor mixture. The reactor was approximately 80% full at the reaction temperatures utilized. The back pressure control valve was set for 50 psig. The total hydrogen evolution corresponded to approximately 100% of theory. Tables I, II and III exemplify the controlled temperature adjustments for the process.

A 150-gram portion of the solid soap product was powdered and melted at 275°C-295°C and the 300°C melt added to 17% HCl over a 5-minute period with stirring and ice water external cooling. The temperature was maintained at 85°C-100°C. The temperature could, of course, be higher if a reflux condenser was provided. The boiling point increased as the brine concentration increased. Separation of the brine and the fatty acid layers was complete without additional layer formation. However, the two layers were allowed to sit in a separatory funnel for 10 min. before drawing off the brine lower layer which was discarded.

The fatty acid layer was then washed twice with water. The acid layer after washing contained 1.24-1.31% water, no chlorine (limit of detection 5 ppm), and no sodium ion. The material balances of the soap plus $H_2$ off were 99-100%. Table IV gives the gas chromatographic analysis of the product formed.

## TABLE I

### RUN 1

| Time Min. | Reaction Temp. °C | Pressure psig | Liters $H_2$ Out |
|---|---|---|---|
| 0 | 83 | 16 | 0 |
| 40 | 220 | 23 | 0 |
| 48 | 242 | 41 | 0.8 |
| 62 | 260 | 50 | 9.0 |
| 77 | 264 | 50 | 17 |
| 85 | 262 | 51 | 22 |
| 99 | 261 | 51 | 31 |
| 110 | 262 | 52 | 37 |
| 130 | 265 | 52 | 49 |
| 150 | 271 | 53 | 64 |
| 180 | 280 | 53 | 104 |
| 217 | 282 | 54 | 127 |
| 234 | 286 | 54 | 141 |
| 269 | 300 | 50 | 160 |
| 284 | 302 | 50 | 161 |

In all, 163.5 liters of $H_2$ gas was vented.
There was no evidence of foaming or expansion.

## TABLE II

### RUN 2

| Time Min. | Reaction Temp. $^{\circ}$C | Pressure psig | Liters $H_2$ Out |
|---|---|---|---|
| 0 | 82 | 20 | 0 |
| 12 | 138 | 21 | 0 |
| 32 | 202 | 25 | 0 |
| 41 | 228 | 31 | 0 |
| 50 | 239 | 40 | 0 |
| 55 | 241 | 47 | 0 |
| 57 | 242 | 50 | 0 |
| 61 | 244 | 56 | 0 |
| 70 | 249 | 52 | 1.7 |
| 78 | 252 | 52 | 2.5 |
| 97 | 260 | 52 | 7 |
| 107 | 266 | 52 | 12 |
| 113 | 266 | 55 | 15 |
| 125 | 266 | 55 | 21 |
| 140 | 267 | 55 | 33 |
| 148 | 266 | 55 | 36 |
| 173 | 264 | 55 | 48 |
| 187 | 264 | 55 | 57 |
| 210 | 270 | 56 | 69 |
| 225 | 274 | 56 | 80 |
| 231 | 278 | 56 | 90 |
| 248 | 284 | 56 | 106 |
| 261 | 288 | 56 | 117 |
| 270 | 291 | 56 | 123 |
| 277 | 293 | 57 | 130 |
| 290 | 304 | 57.5 | 141 |
| 308 | 309 | 57 | 153 |
| 310 | 306 | 56 | 156.5 |

In all, 161.5 liters of $H_2$ gas was vented.
There was no evidence of foaming or expansion.

## TABLE III

### RUN 3

| Time Min. | Reaction Temp. $^{O}C$ | Pressure psig | Liters $H_2$ Out |
|---|---|---|---|
| 0 | 50 | 10 | 0 |
| 21 | 160 | 10 | 0 |
| 35 | 210 | 15 | 0 |
| 45 | 238 | 28 | 0 |
| 50 | 250 | 41 | 0 |
| 55 | 253 | 50 | 0.25 |
| 69 | 251 | 50 | 4 |
| 75 | 260 | 51 | 6 |
| 85 | 262 | 53 | 14 |
| 102 | 265 | 55 | 18 |
| 130 | 266 | 55 | 33.5 |
| 145 | 266 | 55 | 42 |
| 157 | 267 | 56 | 48 |
| 167 | 268 | 56 | 54.5 |
| 184 | 270 | 56 | 66 |
| 198 | 274 | 57 | 75 |
| 222 | 279 | 56 | 94 |
| 243 | 284 | 56 | 111 |
| 262 | 291 | 56 | 125 |
| 307 | 298 | 52 | 157 |
| 327 | 294 | 52 | 159 |

In all, 162 liters of $H_2$ gas was vented
There was no evidence of foaming or expansion.

## TABLE IV

| 7911 Acid Material | Run 1 | Run 2 | Run 3 |
|---|---|---|---|
| % $C_7$ acids, total | 34.3 | 31.9 | 30.4 |
| branched | 8.8 | 7.9 | 7.7 |
| normal | 25.5 | 24.0 | 22.7 |
| % $C_9$ acids, total | 39.8 | 40.0 | 39.5 |
| branched | 12.4 | 11.8 | 11.5 |
| normal | 27.4 | 28.2 | 28.0 |
| % $C_{11}$ acids, total | 25.7 | 27.5 | 27.4 |
| branched | 8.3 | 9.1 | 9.0 |
| normal | 17.4 | 18.4 | 18.4 |
| High Boilers (dimer alcohol impurities) | 0.2 | 0.2 | 1.9 |
| Total Acids | 99.8 | 99.4 | 97.3 |
| Alcohol | 0.0 | 0.2 | 0.8 |
| Total Branched Acids | 29.5 | 28.8 | 28.2 |
| Total Straight Chain Acids | 70.3 | 70.6 | 69.1 |

EXAMPLE II

A 1-liter magnetic drive stirred Inconel autoclave was charged at 80°C-100°C with 523.2 grams (2.44 moles) Alfol 14 alcohol (assumed molecular. wt. 214 gm) and 111.2 grams (2.78 moles) flake NaOH. Conoco's brand Alfol 14 alcohol is a $C_{14}$ primary, straight chain alcohol available from Continental Oil Co. (dodecanol-1 or lauryl alcohol). The autoclave was stirred at 300 rpm with a stirrer. The reactor was previously flushed with nitrogen and then electrically heated. There was no evidence of any foaming during the entire run. The sight glass positioned as in Example I did not show any foaming or expansion in any of the runs. The reactor was estimated to be approx. 80% full at reaction temperature including the added volume of the stirrer. The back pressure control valve was set for approximately either 160 psig or 60 psig. Tables V, VI and VII exemplify the controlled temperature adjustment for the process.

A 50-gram sample of the solid soap product was powdered and melted at 300°C-340°C and the 340°C-350°C melt was added to 15% HCl at 80°C-90°C with stirring and ice water external cooling as needed. The temperature was maintained at 85-100°C to keep the $C_{14}$ acid liquid (the temperature can be higher providing there is a reflux condenser). The boiling point kept rising as the brine concentration increased. The addition of the molten soap took about 5 minutes for 50 grams to be added to 54.4 grams of 15% HCl. There was no unreacted soap left after the molten soap addition. This was checked by analysis of the fatty acid layer. Separation of the brine and the fatty acid layers was complete without any additional layer formation. However, the two layers were allowed to sit in a separatory funnel for about 10 minutes before drawing off the brine lower layer which was discarded. The amount of 15% HCl used provides an aqueous layer with a pH of 2.

The fatty acid layer was then washed twice with water. For the 50 grams of $C_{14}$ soap, 90 grams of water was used for each wash. Layer separation was excellent and rapid.

The $C_{14}$ acid layer after washing contained no chlorine (limit of detection 5 ppm) and no sodium ion.

## TABLE V

### RUN 4

| Time Min. | Reaction Temp. $^{\circ}C$ | Pressure psig | Liters $H_2$ Off |
|---|---|---|---|
| 0 | 84 | 23 | - |
| 10 | 152 | 25 | - |
| 20 | 200 | 28 | - |
| 30 | 238 | 32 | - |
| 40 | 264 | 55 | - |
| 43 | 280 | 80 | - |
| 47 | 288 | 125 | 1 |
| 50 | 300 | 150 | 6.5 |
| 54 | 308 | 150 | 15.5 |
| 60 | 324 | 150 | 29 |
| 68 | 342 | 150 | 38.5 |
| 72 | 350 | 150 | 44 |
| 76 | 355 | 150 | 49 |
| 81 | 358 | 150 | 60 |
| 87 | 358 | 150 | 76 |
| 91 | 360 | 150 | 87 |
| 95 | 364 | 150 | 98.5 |
| 102 | 369 | 151 | 98 |
| 106 | 373 | 150 | 98.5 |
| 135 | 378 | 150 | 99.2 |

There was no evidence of foaming or expansion.

## TABLE VI

### RUN 5

| Time Min. | Reaction Temp. $^{\circ}C$ | Pressure psig | Liters $H_2$ Off |
|---|---|---|---|
| 0 | 136 | 26 | - |
| 32 | 267 | 65 | - |
| 40 | 286 | 158 | - |
| 42 | 291 | 150 | 5 |
| 47 | 301 | 150 | 22 |
| 50 | 316 | 150 | 47 |
| 56 | 331 | 152 | 60 |
| 62 | 348 | 152 | 78 |
| 66 | 354 | 150 | 90 |
| 70 | 364 | 151 | 98 |
| 72 | 367 | 151 | 100 |
| 77 | 370 | 150 | 101.5 |

There was no evidence of foaming or expansion.

## TABLE VII

### RUN 6

| Time Min. | Reaction Temp. °C | Pressure psig | Liters $H_2$ Off |
|---|---|---|---|
| 0 | 100 | 25 | — |
| 12 | 192 | 30 | — |
| 30 | 234 | 32 | — |
| 38 | 292 | 157 | 2 |
| 43 | 308 | 157 | 16 |
| 48 | 310 | 157 | 27 |
| 52 | 326 | 157 | 40 |
| 56 | 338 | 165 | 48 |
| 66 | 364 | 150 | 63 |
| 70 | 368 | 158 | 75 |
| 73 | 370 | 158 | 90 |
| 78 | 370 | 157 | 100 |
| 85 | 370 | 158 | 103 |

There was no evidence of foaming or expansion.

### EXAMPLE III

A 1-liter magnetic drive stirred Inconel autoclave was charged at room temperature with 479.2 grams (3.35 moles) of Conoco's brand Alfol 610 alcohol (assumed molecular wt. 143 grams) and 152.7 grams (3.82 moles) flake NaOH. The Alfol 610 alcohol is comprised of $C_6$, $C_8$ and $C_{10}$ straight chain primary or normal alcohols. The autoclave was stirred at 300 rpm. The reactor was first flushed with nitrogen and then electrically heated with stirring. There was no evidence of any foaming during the runs. A thermocouple probe at the 90% full level and a sight glass just above the reactor but below the condenser did not show any foaming or expansion. The reactor was approximately 80% full at reaction temperature including the added volume of the stirrer. The back pressure control valve was set for approximately 160 psig. The total hydrogen evolution corresponded to approximately 100% of theory. Data in Table III is illustrative of the effects of high temperatures on the reaction product. In Runs 12 and 13, the final reaction product contained about 5.5% cracked hydrocarbon.

A 150-gram amount of the solid soap product was powdered and melted at 295°C-335°C and 150 grams of the 340°C-350°C melt was added over a 3-minute period to 15% HCl with stirring and cooling as needed to maintain a 85°C-100°C temperature. Sufficient HCl is used to provide an aqueous layer having a pH of 2.0-2.5. Unreacted soap was left after separation of the fatty acid from the brine layers. The fatty acid layer was washed twice with water.

## TABLE VIII

### EFFECT OF TEMPERATURE

| Run No. | Temp. °C | Press. psig | Time hrs. | % Alc. Conver- sion (H2 Off) | % Mole Acid Yield | Wt. % High Boilers | Comments |
|---|---|---|---|---|---|---|---|
| 7 | 269-273 | 148-150 | 1.5 | 91 | 93 | 6.5 | |
| 8 | 264-370 | 146-152 | 0.9 | 93 | 94.5 | 5.5 | |
| 9 | 292-370 | 156-158 | 1.0 | 95 | 99 | 1 | |
| 10 | 275-370 | 60-128 | 1.1 | 94 | 95 | 5 | |
| 11 | 278-365 | 65-70 | 1.1 | 100 | 96 | 3.5 | |
| 12 | 265-488 | 162-190 | 1.1 | 90 | 88 | 12 | }5.5% |
| 13 | 269-450 | 150-203 | 2.1 | 91.5 | 82.5 | 12 | }cracked }products |

TABLE IV

A 1-liter magnetic drive stirred Inconel autoclave was charged with 479.2 grams (3.35 moles) of Conoco's brand Alfol 610 alcohol (assumed molecular weight = 143) and 152.7 grams of NaOH (3.82 moles = 13.8 mole % excess). The autoclave was stirred with an anchor shaped stirrer at 300 rpm. The reactor was first flushed with nitrogen and then heated with stirring. After 1 hour, the temperature had reached 230°C. The pressure was 40 psig. After 90 minutes, the temperature was 270°C and the pressure had reached 163 psig. A pressure relief device preset at 165-170 psig then began to open intermittently to maintain the pressure at the preset level. The temperature was gradually raised in accordance with a - schedule to maintain the contents of the autoclave liquid. After 107 minutes, a total of 18 liters of gas at atmospheric pressure had passed out the relief device. The gas volume was measured by a gas meter. The temperature was then 285°C. After 116 minutes, the temperature was 296°C and total gas out was 33 liters. After 126 minutes, the temperature was 303°C and gas volume out was 60 liters. After 131 minutes, the temperature was 315°C and gas volume 75 liters. After 139 minutes, the temperature was 347°C and gas volume 120 liters. After 147 minutes, the temperature was 369°C and gas volume was 152 liters. After another 10 minutes at this temperature, only a total of 152.3 liters of gas had been vented out. A calculation of the gas remaining in the autoclave at this temperature and at the pressure of 168 psig converted to atmospheric pressure (corrected for the nitrogen at the start) showed that a total of 163 liters of hydrogen was formed. Theory is 163.5 liters. The hot molten soaps (sodium carboxylates) were emptied out from the bottom of the autoclave through a heated valve into a beaker containing an excess of well-stirred 15% HCl. The temperature was easily maintained at 25-35°C by external wet ice cooling. At this concentration of HCl, the NaCl formed remained in solution. The fatty acid formed a separate liquid (oil) phase. After all the molten soap was added to the dilute hydrochloric acid, the two liquid layers were easily separated and the organic layer was given a water wash. No foaming or emulsions were encountered in the acidification or water washing steps. The organic layer comprising a mixture of straight chain $C_6$, $C_8$ and $C_{10}$ acids had weight 526 grams. A gas chromatographic analysis showed the product to be 98.8% $C_6$, $C_8$ and $C_{10}$ straight chain acids and 1.1% high boiling acids. A mass spectrographic analysis showed these to be dimeric acids ($C_{12}$, $C_{14}$, $C_{16}$, $C_{18}$ and $C_{20}$ acids) and $C_{13}$ nuclear magnetic resonance analysis showed these acids to have a simple branch at the $C_2$ (alpha) position. Thus, the yield of $C_6$, $C_8$ and $C_{10}$ acids was 99 mole %. The dimeric acids could be readily separated if desired from the other lower molecular weight acids by distillation.

## Claims

1. A process for preparing fatty acids comprising the oxidative-dehydrogenation of at least one oxygen-containing compound with an alkali metal hydroxide at a sufficient temperature to produce the salt of the acid while avoiding substantial by-product formation, then contacting the salt with a mineral acid to form the fatty acid, and wherein the temperature during the reaction is gradually increased so as to maintain the reaction medium in a liquid non-viscous state.

2. A process as claimed in claim 1 characterised in that the temperature during the process is maintained at from about 5°C to about 10°C above the melting point of the reaction medium.

3. A process as claimed in claim 1 or claim 2 characterised in that the oxygen-containing compound is a compound wherein at least one oxygen atom is attached to a carbon atom having at least two substituent hydrogen atoms.

4. A process as claimed in claim 3 characterised in that the oxygen-containing compound is selected from the group consisting of branched and straight chained primary alcohols, glycols, esters, ethers and aldehydes.

5. A process as claimed in claim 4 characterised in that the oxygen-containing compound is a branched or straight chained primary alcohol or mixture thereof.

6. A process as claimed in claim 4 characterised in that more than one oxygen-containing compound is reacted with the alkali metal compound.

7. A process as claimed in any of claims 1 to 6 characterised in that the oxygen-containing compound has from 1 to 40 carbon atoms per molecule.

8. A process as claimed in any of claims 1 to 7 characterised in that the alkali metal hydroxide is used in an amount of from 3% to about 20% in excess of stoichiometric quantities.

9. A process as claimed in any of claims 1 to 8 characterised in that the alkali metal hydroxide is potassium hydroxide or sodium hydroxide.

10. A process as claimed in any of claims 1 to 9 characterised in that the mineral acid is hyrochloric acid.

11. A process as claimed in any of claims 1 to 10 characterised in that the alkali metal hydroxide/oxygen-containing compound reaction product is maintained as a liquid throughout the entire reaction by gradually increasing the reaction temperature during the reaction process.

12. A process as claimed in any of claims 1 to 11 characterised in that the salt of the acid after formation is maintained in a molten state before addition to the mineral acid.

13. A process as claimed in any of claims 1 to 12 characterised in that water formed during the process is vented form the reactor.

14. A process as claimed in any of claims 1 to 13 characterised in that the temperature of the process ranges from about 175°C to about 400°C.